# EUROPEAN PATENT SPECIFICATION

(11) **EP 4 389 054 B1**
(45) Date of publication and mention of the grant of the patent: **10.09.2025**
(21) Application number: 24167106.4
(22) Date of filing: 02.06.2021
(51) Int. Cl.: A61B 17/68, A61B 34/30, A61B 17/16, A61B 17/70, A61B 17/32, A61B 17/86, A61B 17/88, A61B 90/00, A61B 34/37, A61B 34/00, A61B 17/00

(54) **OPERATION SYSTEM FOR BONE TISSUE**
BETRIEBSSYSTEM FÜR KNOCHENGEWEBE
SYSTÈME D'OPÉRATION POUR TISSU OSSEUX

(43) Date of publication of application: 26.06.2024
(62) Divisional of application: 21925118.8
(73) Proprietor: Shanghai Droidsurg Medical Co., Ltd, Shanghai 201612 (CN)
(72) Inventor: WANG, Shaobai, Shanghai, 201612 (CN)
(74) Representative: Bayramoglu et al.

(56) References cited:
- EP-A1- 3 123 970
- US-A1- 2005 038 438
- US-A1- 2013 123 847
- US-A1- 2017 258 533
- US-A1- 2017 340 370
- US-A1- 2019 029 736
- US-A1- 2019 201 055
- US-A1- 2020 324 408
- US-B2- 8 529 570

## Description

### TECHNICAL FIELD

The present disclosure relates to the medical field, in particular to an operation method and an operation system for bone tissue.

### BACKGROUND

In some orthopedic operations, medical instruments are required for re-combining two separated vertebral segments for fixation. For example, in the process of treating spinal stenosis, it is required to cut off a target spinal segment before operation treatment, and then fix the two cut spinal segments. In existing operation methods for bone tissue, screws are generally used to fix two spinal segments. Such methods cannot adjust offset of the two spinal segments caused during space formation, such that after direct fixation, there will be offset between the two spinal segments, postoperative risks are likely to occur, or it is necessary to adjust a location between adjacent vertebrae, thereby increasing the operation difficulty, prolonging operation time and improving input cost.

Disclosed in a patent application with Publication No. CN1455656A is a method for spacing a spinal canal, which re-fixes the separated vertebral space. In the above method, an implant including a stent, a washer, a screw and a rope is used, the stent is squeezed into the space, the screw is inserted into the washer and the stent and squeezed into the front of a vertebra for each space of the vertebra, and the rope is connected to the washer at each end and tied to penetrate the back of the vertebra to fix the back of the vertebra to its front, thereby re-stabilizing the cut spinal canal. However, the above method is complicated in operation, and is likely to form significant stress on the spinal canal, which affects postoperative experience of patients. Moreover, since the two spinal segments are fixed with the screws, postoperative risks are likely to occur due to offset between the two spinal segments, or it is necessary to adjust the location between adjacent vertebrae, thereby increasing the operation difficulty, prolonging operation time and improving input cost.

US2019/201055A1 discloses a system that includes a first pedicle screw, a second pedicle screw, and an adjustable rod having an outer housing coupled to one of the first pedicle screw and the second pedicle screw, the outer housing having a threaded shaft secured to one end thereof extending along an interior portion thereof. The system further includes a hollow magnetic assembly disposed within the outer housing and having a magnetic element disposed therein, the hollow magnetic assembly having an internal threaded surface engaged with the threaded shaft, the magnetic assembly being coupled to the other of the first pedicle screw and the second pedicle screw, wherein the hollow magnetic assembly rotates in response to an externally applied magnetic field to thereby lengthen or shorten the distance between the first pedicle screw and the second pedicle screw.

US2017340370A1 discloses an implant assembly that includes a first implant having a body with a first end and a second end and a hollow passage extending therethrough between the first end and the second end. The first implant is sized for insertion into a first bone segment. A connector has a first end rotatably connected to the first implant at the first end of the first implant, a second end insertable into a second bone segment, and a connector body extending through the hollow passage of the first connector. A method of inserting the implant assembly is also provided.

US2013/123847A1 discloses an implant for expanding a spinal canal having an upper portion, a lower portion, and an inner member. The inner member communicates with a swivelable coupling located at each end of the inner member within the implant. Each swivelable coupling also interacts with a respective one of the upper and lower portion, within an inner bore thereof. Accordingly, movement of the inner member relative to one or both of the upper and the lower portions, via one or both swivelable couplings, translates the upper portion away from the lower portion, about a vertebral cut, to widen the vertebral cut and expand the spinal canal. The swivelable action of the couplings during translation allows angulation of the inner member, relative to a longitudinal axis of the implant, to accommodate a natural lateral shift occurring during a widening of a vertebral cut.

EP3123970A1 discloses an implant that enhances structural stability across a joint between a first and a second bone to be immobilized. The implant comprises a first and a second element, wherein the first element comprises a proximal, a distal and an intermediate section. The front end of the implant is formed as a pointed head with structures enabling the implant to penetrate bone material, including a channel-like structure running from the front end to a reservoir in the intermediate section. The reservoir is provided for receiving bone material through the channel-like structure and distributing it into a space between two bones upon placing the implant into the bones. A tool is provided for placing the implant into two bones, wherein the tool has means for connecting it to structures in the first element and the second element, so that the two elements can be moved independently.

US2019/029736A1 discloses a surgical instrument that comprises a first member including a drive engageable with a first mating surface of a bone fastener. A second member is rotatable relative to the first member and includes an element engageable with a second mating surface of the bone fastener. An actuator is connected with the second member and includes visual indicia of engagement of the element with the second mating surface. Systems, spinal implants and methods are disclosed.

US2005038438A1 discloses a bone screw that includes a shank having a threaded proximal portion and a distal portion with a major diameter that is less than a minor diameter of the threaded proximal portion. A transitional region of decreasing diameter can be disposed between the threaded proximal portion and the distal portion, and a driver receiving element is preferably disposed on a proximal end of the bone screw. **In** use, the proximal and distal portions of the bone screw are adapted to engage two segments of bone, and to create a distraction force between the two segments of bone.

US8529570B2 discloses a technique for performing laminar osteotomy (dividing of the spinal lamina, a posterior structure of vertebra). The term osteotomy is derived from os(bone), tomy(to cut or remove), and laminoplasty (the reshaping of the lamina). This technique can be performed with or without visual assistance such as cameras used with endoscopy, or image guidance such as fluoroscopy, computer tomography (CT) or magnetic resonance imaging (MRI).

To this end, it is necessary to develop a novel operation method and an operation system for bone tissue, so as to solve the above problems in the prior art.

### SUMMARY

An objective of the present disclosure is to provide an operation system for bone tissue, so to solve the problem that an operation system for bone tissue in the prior art is complicated in operation, significant stress is likely to form on a spinal canal, postoperative experience of patients are affected consequently, in addition, postoperative risks are likely to occur due to offset between two spinal segments, or it is necessary to adjust a location between adjacent vertebrae.

To achieve the above objective, the present disclosure provides an operation system for bone tissue as defined in the appended claims.

The operation system for bone tissue of the present disclosure has the beneficial effects: the operation system for bone tissue includes the first implantation device and the second implantation device, where the first implantation device includes the hollowed structure, and the adapter structure is arranged in the hollowed structure, such that the first implantation device may be used by a user in hand or by means of a robot, etc. to penetrate the first vertebral segment, and the hollowed structure is caused to face the end face of the second vertebral segment and make surface contact with the second vertebral segment to perform a location step, such that damage to a post-osteotomy section may be avoided, the first vertebral segment and the second vertebral segment may be pre-located by means of the first implantation device, and the first vertebral segment and the second vertebral segment separated by the space may pre-adapt to adjacent vertebrae thereof correspondingly by implanting the first implantation device; and at least the portion of the outer side wall of the bottom of the second implantation device is provided with the corresponding adapter structure to be fixedly connected to the adapter structure, such that the second implantation device may be used by the user in hand or by means of the operation robot, etc. to penetrate the hollowed structure to be fixedly connected to the second vertebral segment, the second implantation device is caused to be fixedly connected to the hollowed structure by means of fixed connection between the corresponding adapter structure and the adapter structure to perform a fixation step, that is, while the first vertebral segment and the second vertebral segment separated by the space are relatively re-fixed by means of interaction between the second implantation device and the first implantation device, the stress is reduced, postoperative experience of patients are prevented from being influenced, and the second vertebral segment may adapt to adjacent vertebrae thereof, thereby directly adjusting the location between the second vertebral segment and the adjacent vertebrae, avoiding special re-adjustment of the location between the adjacent vertebrae, shortening operation time, eliminating demand for auxiliary adjustment instruments and reducing cost input.

The operation system for bone tissue has the beneficial effects as follows: accurate control is facilitated to a higher extent and higher operation flexibility is achieved.

The operation system for bone tissue has the following beneficial effect as follows: accuracy of an osteotomy process is ensured, risks of nerve and blood vessel damage due to overshooting during osteotomy is reduced.

### BRIEF DESCRIPTION OF DRAWINGS

Fig. 1 is a flowchart of an operation method for bone tissue in an embodiment not part of the present invention;
Fig. 2 is a structural block diagram of a first operation system for bone tissue in an embodiment not part of the present invention;
Fig. 3 is a structural schematic diagram for fixing a broken vertebral pedicle with an implantation device in some embodiments not part of the present invention;
Fig. 4 is a structural block diagram of a second operation system for bone tissue in an embodiment not part of the present invention;
Fig. 5 is a structural block diagram of a third operation system for bone tissue in an embodiment not part of the present invention;
Fig. 6 is a structural schematic diagram showing a nut structure acting on a first vertebral segment in some embodiments not part of the present invention;
Fig. 7 is an enlarged structural schematic diagram of portion A shown in Fig. 6;
Fig. 8 is a schematic diagram of the nut structure shown in Fig. 6;
Fig. 9 is a structural schematic diagram of a bottom of the nut structure shown in Fig. 8;
Fig. 10 is a schematic diagram of an operation state in which an implantation mechanical arm clamps the nut structure in some embodiments of the present invention;
Fig. 11 is an enlarged structural schematic diagram of portion B shown in Fig. 10;
Fig. 12 is a structural schematic diagram showing a screw structure acting on the nut structure and a second vertebral segment in some embodiments of the present invention;
Fig. 13 is a schematic diagram of the screw structure shown in Fig. 12;
Fig. 14 is a structural block diagram of an operation system for bone tissue in an embodiment of the present invention;
Fig. 15 is a structural schematic diagram of an osteotomy device in an embodiment of the present invention;
Fig. 16 is a sectional view of a portion of the structure shown in Fig. 15;
Fig. 17 is a partial structural schematic diagram of a buffer drive portion shown in Fig. 15; and
Fig. 18 is a structural schematic diagram of another osteotomy device in an embodiment of the present invention.

### DETAILED DESCRIPTION OF THE EMBODIMENTS

To make objectives, technical solutions, and advantages of embodiments of the present disclosure clearer, the technical solutions in the embodiments of the present disclosure will be clearly and completely described. Apparently, the described embodiments are some rather than all of the embodiments of the present disclosure. Based on the embodiments of the present disclosure, all the other embodiments obtained by those of ordinary skill in the art without inventive effort are within the scope of the present disclosure. Unless otherwise defined, technical terms or scientific terms used herein should have ordinary meanings understood by those with ordinary skills in the field to which the present disclosure belongs. As used herein, similar words such as "include" mean that elements or objects appearing before the word cover elements or objects listed after the word and their equivalents, but do not exclude other elements or objects.

The embodiments of the present disclosure provide an operation system for bone tissue, so to solve the problem that an operation system for bone tissue in the prior art is complicated in operation, significant stress is likely to form on a spinal canal, postoperative experience of patients are affected consequently, in addition, postoperative risks are likely to occur due to offset between two spinal segments, or it is necessary to adjust a location between adjacent vertebrae.

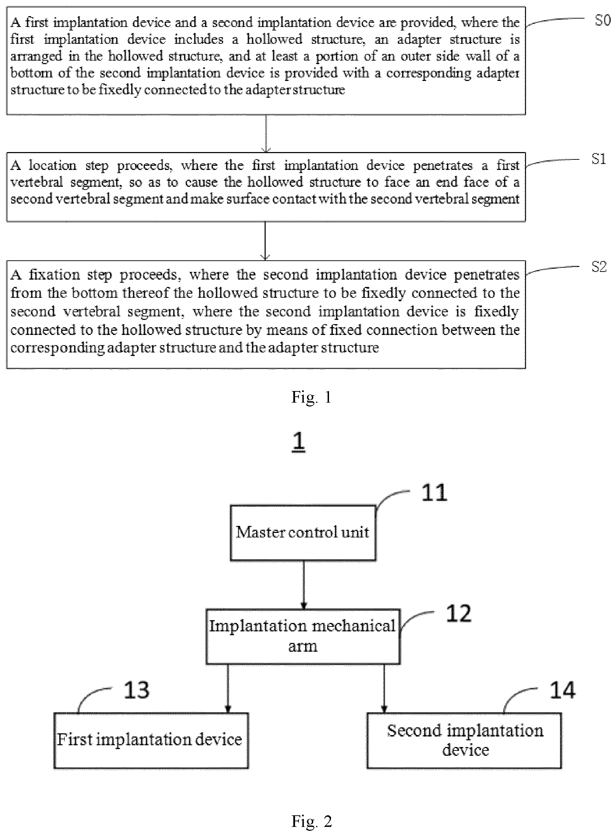

Fig. 1 is a flowchart of the operation method for bone tissue not part of the present invention.

With reference to Fig. 1, the operation method for bone tissue includes:
S0: a first implantation device and a second implantation device are provided, where the first implantation device includes a hollowed structure, an adapter structure is arranged in the hollowed structure, and at least a portion of an outer side wall of a bottom of the second implantation device is provided with a corresponding adapter structure to be fixedly connected to the adapter structure;
S1: a location step proceeds, where the first implantation device penetrates a first vertebral segment, so as to cause the hollowed structure to face an end face of a second vertebral segment and make surface contact with the second vertebral segment; and
S2: a fixation step proceeds, where the second implantation device penetrates from the bottom thereof the hollowed structure to be fixedly connected to the second vertebral segment, the second implantation device is fixedly connected to the hollowed structure by means of fixed connection between the corresponding adapter structure and the adapter structure.

Before the location step in S1 is performed, an osteotomy device is used to act on target bone tissue to form a space, the operation method for bone tissue acts on vertebrae with a space, and the space penetrates at least one vertebral pedicle, and one vertebral segment, including a lamina, of the two vertebral segments separated by the space is the first vertebral segment and a vertebral segment, including a vertebral body, of the two vertebral segments separated by the space is the second vertebral segment.

In some embodiments not part of the present invention, the operation method for bone tissue further includes a vertebral segment spacing step after the location step in S1 and before the fixation step in S2, where the vertebral segment spacing step includes: a moving distance of the first implantation device towards the second vertebral segment is adjusted, the hollowed structure is caused to move towards the second vertebral segment, and a vertebral segment distance between the first vertebral segment and the second vertebral segment is spaced. The vertebral segment distance between the first vertebral segment and the second vertebral segment is controlled in the vertebral segment spacing step, and the vertebral segment distance is planned by a doctor according to an image result of a patient with a goal of spacing by a vertebral foramen space and reducing compression.

In some embodiments not part of the present invention, in the location step in S1, the step of causing the first implantation device to penetrate a first vertebral segment includes: the first implantation device is used to move from a location, near a transverse process, of the lamina in a direction towards the space until the first implantation device penetrates the first vertebral segment.

The operation system for bone tissue includes a first implantation device and a second implantation device, where the first implantation device includes a hollowed structure, an adapter structure is arranged in the hollowed structure, and at least a portion of an outer side wall of a bottom of the second implantation device is provided with a corresponding adapter structure to be fixedly connected to the adapter structure.

In some embodiments, a user holds the first implantation device to penetrate the first vertebral segment, and the hollowed structure is caused to face an end face of the second vertebral segment and make surface contact with the second vertebral segment to perform a location step. The user further holds the second implantation device to penetrate the hollowed structure and then to be fixedly connected to the second vertebral segment, and the second implantation device is caused to be fixedly connected to the hollowed structure by means of fixed connection between the corresponding adapter structure and the adapter structure to perform a fixation step.

In some other embodiments, the operation system for bone tissue further includes an operation robot, the operation robot is used for causing the first implantation device to penetrate the first vertebral segment, and causing the hollowed structure to face the end face of the second vertebral segment and make surface contact with the second vertebral segment to perform the location step. The operation robot is further used for causing the second implantation device to penetrate the hollowed structure, and then be fixed connected to the second implantation device, and causing the second implantation device to be fixedly connected to the hollowed structure by means of fixed connection between the corresponding adapter structure and the adapter structure to perform the fixation step.

An operation system for bone tissue is provided for performing the operation method for bone tissue. The operation system for bone tissue includes a first implantation device, a second implantation device and an operation robot. The first implantation device includes a hollowed structure, an adapter structure is arranged in the hollowed structure, and at least a portion of an outer side wall of a bottom of the second implantation device is provided with a corresponding adapter structure to be fixedly connected to the adapter structure. The operation robot is used for causing the first implantation device to penetrate the first vertebral segment, and causing the hollowed structure to face an end face of the second vertebral segment and make surface contact with the second vertebral segment to perform a location step. The operation robot is further used for causing the second implantation device to penetrate the hollowed structure, and then be fixed connected to the second implantation device, and causing the second implantation device to be fixedly connected to the hollowed structure by means of fixed connection between the corresponding adapter structure and the adapter structure to perform a fixation step.

The operation system for bone tissue further includes a master control unit and an implantation mechanical arm, the master control unit being in communication with the implantation mechanical arm; where the implantation mechanical arm is provided with a clamping structure to adapt to the first implantation device and the second implantation device; and the master control unit controls the implantation mechanical arm to clamp the first implantation device to penetrate a first vertebral segment and to be fixedly connected to same, so as to form a spacing distance between the first vertebral segment and a second vertebral segment, and further the master control unit controls the implantation mechanical arm to clamp the second implantation device to penetrate the first implantation device and to be fixedly connected to same and the second vertebral segment.

The operation robot includes a master control unit and an implantation mechanical arm, and the implantation mechanical arm, the first implantation device and the second implantation device are all in communication connection with the master control unit. The implantation mechanical arm is provided with a clamping structure to adapt to the first implantation device and the second implantation device; and the master control unit controls the implantation mechanical arm to clamp the first implantation device to penetrate a first vertebral segment and to be fixedly connected to same, so as to form a spacing distance between the first vertebral segment and a second vertebral segment, and further the master control unit controls the implantation mechanical arm to clamp the second implantation device to penetrate the first implantation device and to be fixedly connected to same and the second vertebral segment.

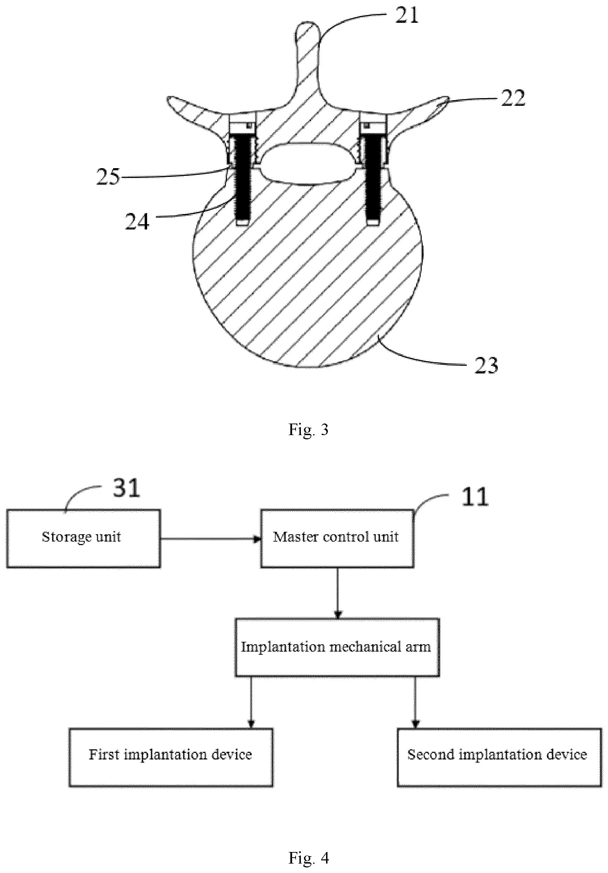

Fig. 2 is a structural block diagram of a first operation system for bone tissue in an embodiment not part of the present invention, and Fig. 3 is a structural schematic diagram for fixing a broken vertebral pedicle with an implantation device not part of the present invention.

With reference to Figs. 2 and 3, the operation system for bone tissue 1 shown in Fig. 2 includes a master control unit 11 and an implantation mechanical arm 12, a first implantation device 13 and a second implantation device 14 which are communication connection with the master control unit 11. The first implantation device 13 and the second implantation device 14 constitute an implantation device 24. A first vertebral segment 22 and a second vertebral segment 23 are separated by a space 25.

Not part of the present invention, the master control unit 11 controls the implantation mechanical arm 12 to clamp the first implantation device 13 to penetrate the first vertebral segment 22 and to be fixedly connected to same, so as to form a spacing distance between the first vertebral segment 22 and the second vertebral segment 23 and guarantee accuracy of the spacing distance, and further the master control unit controls the implantation mechanical arm 12 to clamp the second implantation device 14 to penetrate the first implantation device 13 and to be fixedly connected to same and the second vertebral segment 23.

In some embodiments not part of the present invention, the implantation mechanical arm is provided with a clamping structure to adapt to the first implantation device and the second implantation device.

In some embodiments not part of the present invention, before the location step is performed, the method further includes a preoperative path planning step. The preoperative path planning step includes:
three-dimensional model data and vertebral segment distance requirement data of target bone tissue are provided;
section data are constructed according to the three-dimensional model data;
second vertebral segment section contact data are formed according to the vertebral segment distance requirement data and the section data; and
first vertebral segment implantation path data are formed according to the second vertebral segment section contact data.

Fig. 4 is a structural block diagram of a second operation system for bone tissue not part of the present invention.

With reference to Figs. 2 and 4, the operation system for bone tissue shown in Fig. 4 differs from the operation system for bone tissue 1 shown in Fig. 2 in that the operation system for bone tissue shown in Fig. 4 further includes a storage portion 31 in communication connection with the master control unit 11, and the storage portion 31 stores spacing distance range reference data. The master control unit 11 calls the spacing distance range reference data from the storage unit 31 to control the spacing distance to conform to the spacing distance range reference data, so as to guarantee accuracy of the actual spacing distance.

In some embodiments not part of the present invention, the master control unit 11 stores the spacing distance range reference data, that is, the storage unit 31 is a constituent structure in the master control unit 11.

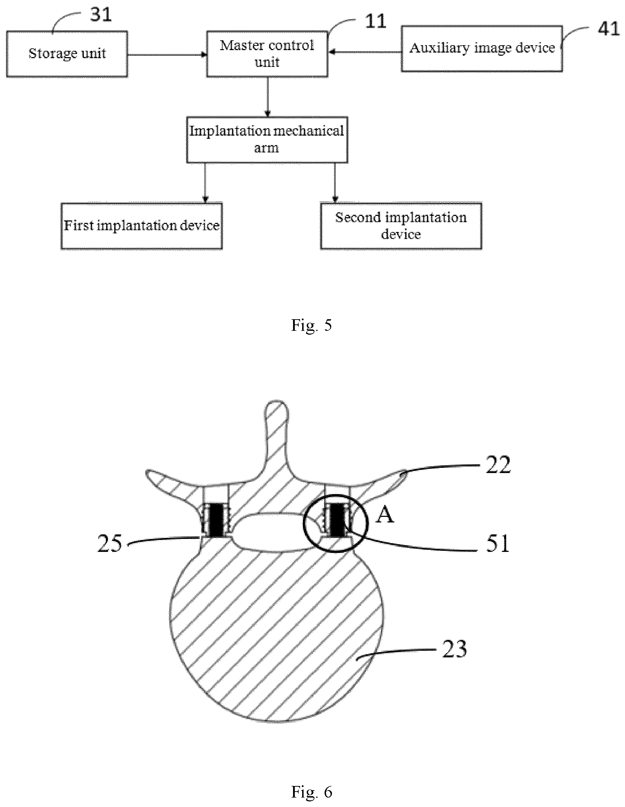

Fig. 5 is a structural block diagram of a third operation system for bone tissue in an embodiment not part of the present invention.

Not part of the present invention, with reference to Figs. 4 and 5, the operation system for bone tissue shown in Fig. 5 differs from that shown in Fig. 4 in that the operation system for bone tissue shown in Fig. 5 further includes an auxiliary image device 41 in communication connection with the master control unit 11.

Not part of the present invention, with reference to Figs. 3 and 5, the auxiliary image device 41 acquires real-time location information of the first vertebral segment 22 and the second vertebral segment 23 and feeds same back to the master control unit 11. The master control unit 11 determines whether the spacing distance conforms to the spacing distance range reference data according to the real-time location information, thus guaranteeing the accuracy of the spacing distance.

In some embodiments not part of the present invention, the auxiliary image device 41 is a C-arm X-ray machine or a computed tomography (CT) apparatus to acquire image information of target bone tissue.

In some embodiments, during movement of the hollowed structure towards the second vertebral segment, the hollowed structure is controlled to face the end face of the second vertebral segment and keep surface contact with the second vertebral segment, thereby avoiding damage to a post-osteotomy section.

In some embodiments, in the location step in S1, the hollowed structure is caused to face the end face of the second vertebral segment and make planar contact or spherical contact with the second vertebral segment, thereby avoiding damage to the post-osteotomy section.

In some embodiments not part of the present invention, the first implantation device includes a nut structure provided with internal threads and external nut threads, the second implantation device includes a screw structure provided with external threads, and the external threads adapt to the internal threads.
In some embodiments not part of the present invention, a bottom of the nut structure includes a nut contact surface, such that in the location step in S1, the hollowed structure is caused to face the end face of the second vertebral segment and make planar contact or spherical contact with the second vertebral segment, thereby avoiding damage to the post-osteotomy section.

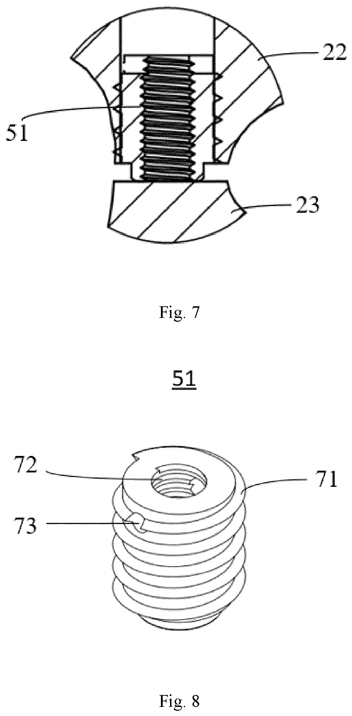

Fig. 6 is a structural schematic diagram showing a nut structure acting on a first vertebral segment in some embodiments not part of the present invention, and Fig. 7 is an enlarged structural schematic diagram of portion A shown in Fig. 6.

With reference to Figs. 2, 6 and 7, the master control unit 11 controls, by means of the implantation mechanical arm 12, the nut structure 51 to penetrate the first vertebral segment 22, and pass through space 25 to make surface contact with the second vertebral segment 23 to form the spacing distance and be fixedly connected to the first vertebral segment 22. The bottom of the nut structure 51 makes surface contact with a top surface of the second vertebral segment 23. By controlling a depth of the nut structure 51 into the first vertebral segment 22, the spacing distance between the first vertebral segment 22 and the second vertebral segment 23, that is, the vertebral segment distance between the first vertebral segment 22 and the second vertebral segment 23, is controlled.

In some embodiments, the spacing distance between the first vertebral segment 22 and the second vertebral segment 23 is an average of several vertical distances from a bottom of the first vertebral segment 22 to the top surface of the second vertebral segment 23.

In some embodiments not part of the present invention, the clamping structure of the implantation mechanical arm includes a casing structure, a stretchable structure accommodated in the casing structure and a lock structure in movable contact with the stretchable structure, the casing structure being detachably and fixedly connected to the first implantation device, and the stretchable structure stretching out to push out the lock structure to abut against an inner wall of the first implantation device, such that the clamping structure of the implantation mechanical arm may be detachably and fixedly connected to the first implantation device, movement of the first implantation device may be conveniently controlled by means of the clamping structure of the implantation mechanical arm in a more accurate manner, which is conducive to movement stability of the first implantation device during implantation.

In some embodiments not part of the present invention, a top of the first implantation device is provided with an accommodation structure, at least a portion of the lock structure pushed out as the stretchable structure stretches out is accommodated in the accommodation structure, the accommodation structure is arranged to accommodate the lock structure, such that firmness of abutting connection between the lock structure and the first implantation device may be better guaranteed, the clamping structure of the implantation mechanical arm may be connected and fixed to the first implantation device more conveniently, and subsequent operations may be facilitated accordingly.

In some embodiments not part of the present invention, the clamping structure of the implantation mechanical arm further includes an electromagnetic control portion electrically connected to the stretchable structure, and the electromagnetic control portion controls the lock structure to enter or exit the accommodation structure by controlling the stretchable structure to move towards or away from the first implantation device, such that accurate control is facilitated to a higher extent and higher operation flexibility is achieved.

In some embodiment not part of the present invention, the lock structure includes several ball structure relatively attracted to two sides of the stretchable structure. When the electromagnetic control portion controls the stretchable structure to move towards the first implantation device, a portion of the stretchable structure is accommodated in the top of the first implantation device, and the several ball structures enter the accommodation structure from the two sides of the stretchable structure and contact with the stretchable structure to compress the stretchable structure.

In some embodiments not part of the present invention, the accommodation structure is arranged in a radial direction of the first implantation device, and an axial length inner diameter of the accommodation structure is smaller than a maximum axial length of the lock structure, such that firmness of abutting connection between the lock structure and the first implantation device may be better guaranteed, the clamping structure of the implantation mechanical arm may be connected and fixed to the first implantation device more conveniently, and subsequent operations may be facilitated accordingly.

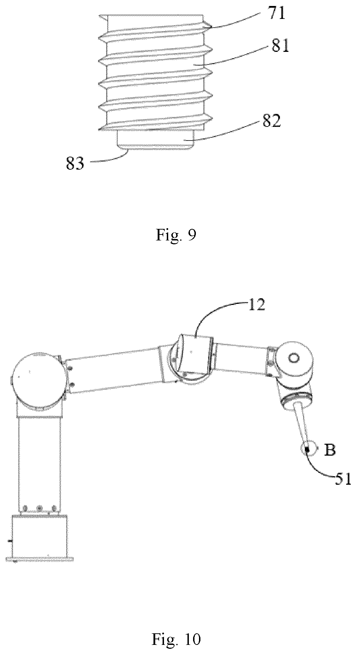

Fig. 8 is a schematic diagram of the nut structure shown in Fig. 6 and Fig. 9 is a structural schematic diagram of a bottom of the nut structure shown in Fig. 8 not part of the present invention.

With reference to Figs. 7-9, an outer side wall of a nut body 81 of the nut structure 51 is provided with nut external threads 71, which is conducive to fixed connection between the nut structure 51 and the first vertebral segment 22 after the expansion distance is formed.

An inner side wall of the nut structure 51 is provided with internal threads 72 to adapt to the second implantation device.

A top of the nut structure 51 is provided with an accommodation structure 73 to adapt to the implantation mechanical arm 12.

A free end of the nut structure 51 converges towards an axis (not shown in the figure) of the nut structure to cause a portion of an outer side wall of the nut structure 51 to be arc-shaped, thereby reducing a friction effect of the surface contact between the nut structure 51 and the second vertebral segment 23.

Specifically, the bottom of the nut body 81 includes a boss 82, an outer diameter of the boss 82 is smaller than an outer diameter of the nut body 81, and a free end edge of the boss 82 is provided with a rounded structure 83, so as to cause the portion of the outer side wall of the nut structure 51 to be arc-shaped, not part of the present invention.

In some embodiments not part of the present invention, a height of the boss 82 is the expansion distance.

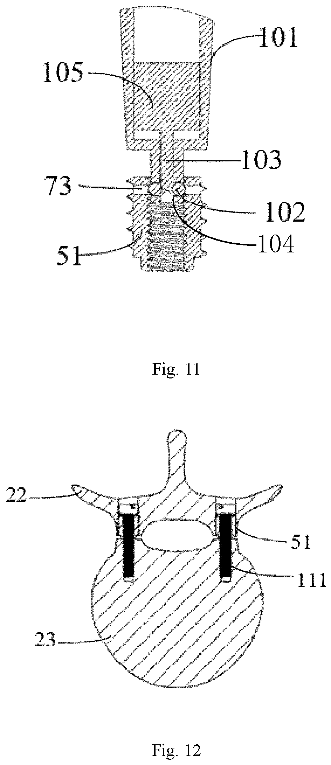

Fig. 10 is a schematic diagram of an operation state in which an implantation mechanical arm clamps the nut structure. Fig. 11 is an enlarged structural schematic diagram of portion B shown in Fig. 10 not part of the present invention.

Not part of the present invention, with reference to Figs. 10 and 11, the implantation mechanical arm 12 is a six-axis mechanical arm. The clamping structure of the implantation mechanical arm 12 includes the casing structure 101, the stretchable structure 103 accommodated in the casing structure, and a lock structure 102 in movable contact with the stretchable structure 103. The casing structure 101 is in clearance fit with a cavity of the nut structure 51, that is, the casing structure 101 may be movably inserted into the cavity of the nut structure 51. The casing structure is further internally provided with a channel 104 allowing the stretchable structure 103 to stretch out and draw back, and the top of the nut structure 51 is provided with the accommodation structure 73. The stretchable structure 103 is electrically connected to the electromagnetic control portion 105.

Further, not part of the present invention, with reference to Fig. 11, the accommodation structure 73 is arranged in a radial direction of the nut structure 51. Specifically, an inner diameter of the accommodation structure 73 is smaller than a diameter of the ball structure 102.

In some embodiments not part of the present invention, the stretchable structure 103 and the electromagnetic control portion 105 constitute an electromagnetic bolt.

Specifically, the electromagnetic control portion 105 causes the stretchable structure 103 to be accommodated in the casing structure 101 when not energized, and the two ball structures 102 are attracted to the bottom of the casing structure 101.

When the nut structure 51 needs to be clamped, the bottom of the casing structure 101 is accommodated in the top of the nut structure 51, and after the electromagnetic control portion 105 is energized, the stretchable structure 103 stretches out of the bottom of the casing structure 101 and moves towards the bottom of the nut structure 51 under the action of electromagnetic attraction. Since the inner diameter of the accommodation channel 73 is smaller than the diameter of the ball structure 102, after one portions of the two ball structures 102 are squeezed into the accommodation structure 73, the other portions clamp the stretchable structure 103 on the opposite sides of the stretchable structure 103, thus clamping and fixing the nut structure 51.

In some embodiments not part of the present invention, the accommodation structure penetrates the top of the nut structure, such that a penetrated structure is formed, and a process is simple and machining is more convenient.

In some embodiments not part of the present invention, the second implantation device 14 includes the screw structure.

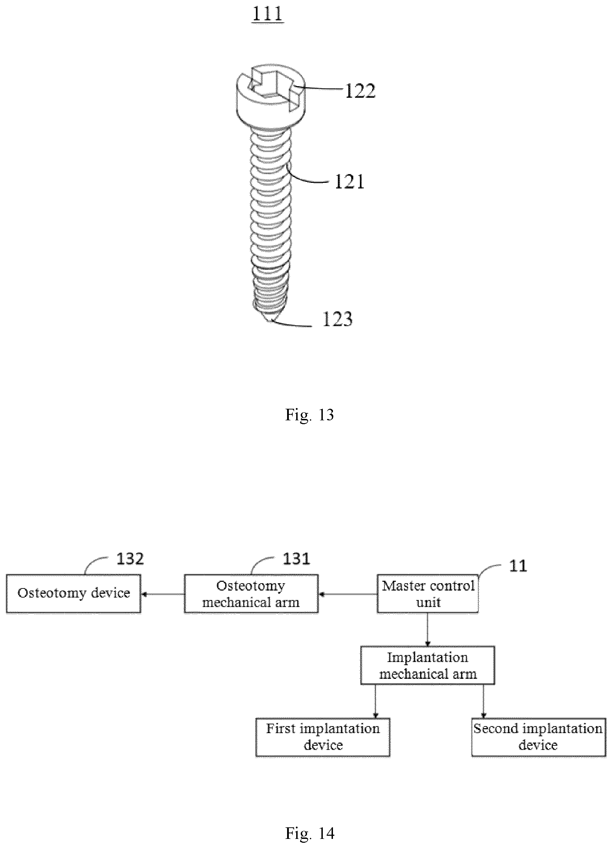

Fig. 12 is a structural schematic diagram for showing a screw structure acting on the nut structure and a second vertebral segment in some embodiments of the present disclosure, and Fig. 13 is a schematic diagram of the screw structure shown in Fig. 12

With reference to Figs. 2, 12 and 13, after the nut structure 51 is implanted into the first vertebral segment 22, the implantation mechanical arm 12 controls the screw structure 111 to be implanted into the second vertebral segment 23 by means of the nut structure 51. The screw structure 111 is provided with the external threads 121 to adapt to the internal threads (not shown in the figure) of the nut structure 51. The screw structure 111 has a bottom in a pointed structure 123, so as to be smoothly screwed into the second vertebral segment 23. By means of the external threads 121, the screw structure 111 may be fixedly connected to the second vertebral segment 23 and the nut structure 51, such that the first vertebral segment 22 and the second vertebral segment 23 may be fixed.

In some embodiments not part of the present invention, with reference to Fig. 13, a top of the screw structure 111 is provided with a screw accommodation structure 122 with the same structure as that of the accommodation structure, so as to accommodate the lock structure and to be detachably and fixedly connected to the casing structure, such that the clamping structure of the implantation mechanical arm may be detachably and fixedly connected to the screw structure, and movement of the screw structure may be conveniently controlled by means of the clamping structure of the implantation mechanical arm in a more accurate manner, which is conducive to movement stability of the screw structure during implantation.

In some embodiments not part of the present invention, before the location step in S1 is performed, an ultrasonic osteotome is used to act on the target bone tissue to form the space.

With reference to Fig. 3 not part of the present invention, after the ultrasonic osteotome is used to form the space 25 shown in Fig. 3 during bone tissue operation, the vertebral pedicle 21 forms the first vertebral segment 22 and the second vertebral segment 23, and then the implantation device 24 penetrates the first vertebral segment 22, and passes through the space 25 to be fixed to the second vertebral segment 23.

However, since there are many nerves and blood vessels around the vertebral pedicle 21, these nerves and blood vessels are needed to be protected from damage during the operation. During osteotomy with the osteotome, at the moment when the vertebral pedicle 21 is cut off, since thrust of the osteotome on bone suddenly disappears, the osteotome may overshoot and continue to move in the direction of action, such that damage to the nerves and the blood vessels and unnecessary damage to patients are likely to occur.

The operation system for bone tissue further includes an osteotomy device for acting on target bone tissue to form a space.

The operation system for bone tissue further includes the master control unit and an osteotomy mechanical arm 131clamping the osteotomy device, where the master control unit is in communication connection with the osteotomy mechanical arm, and the osteotomy device includes an actuator clamped at a free end of the osteotomy mechanical arm, and the master control unit controls the osteotomy mechanical arm 131 to cause the actuator to act on the target bone tissue to form the space.

Fig. 14 is a structural block diagram of an operation system for bone tissue in an embodiment of the present invention.

In some embodiments of the present invention, with reference to Figs. 2, 3 and 14, the operation system for bone tissue shown in Fig. 14 differs from the operation system for bone tissue 1 shown in Fig. 2 is that the operation system for bone tissue shown in Fig. 14 further includes an osteotomy mechanical arm 131 which is in communication connection with a master control unit 11 and holds an osteotomy device 132, and the master control unit 11 controls the osteotomy mechanical arm 131 to cause the osteotomy device 132 to form a space 25 at a vertebral pedicle 21.

The osteotomy device 132 includes an actuator to clamp a free end of the osteotomy mechanical arm 131 and act on target bone tissue.

In some embodiment of the present invention, the actuator includes an ultrasonic osteotome.

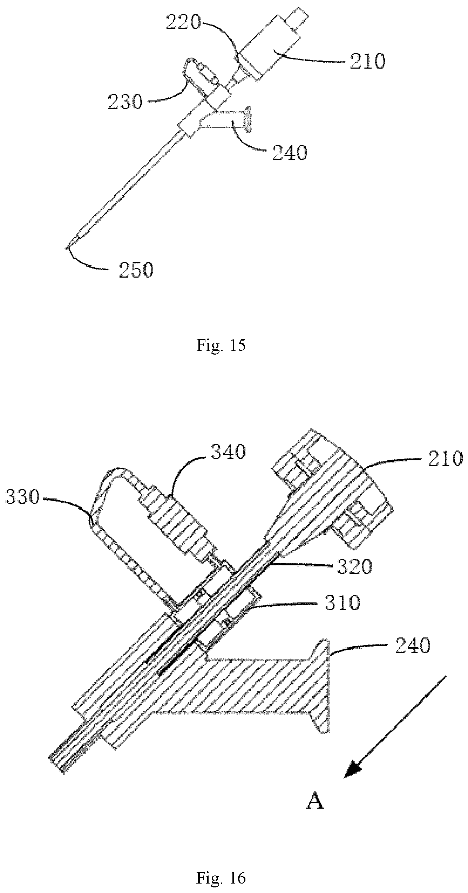

Fig. 15 is a structural schematic diagram of an osteotomy device in an embodiment of the present invention. Fig. 16 is a sectional view of a portion of the structure shown in Fig. 15.

With reference to Figs. 15 and 16, the osteotomy device shown in Fig. 15 includes an actuator 210 and a buffer mechanism (not shown in the figure), and the buffer mechanism (not shown in the figure) partially sleeves the actuator 210. The buffer structure (not shown in the figure) is composed of a pressure sensing portion 220 and a buffer drive portion 230, and the buffer drive portion 230 is fixed to the actuator 210 in a sleeving manner. The pressure sensing portion 220 is arranged on the actuator 210.

The buffer mechanism acts on the actuator 210 according to detected change of the pressure by the actuator 210 acting on the target bone tissue.

With reference to Figs. 15 and 16, the actuator 210 acts on the target bone tissue (not shown in the figure) in the operation direction, that is, an A direction shown in Fig. 16, and the pressure sensing portion 220 detects that the change of the pressure by the actuator 210 acting on the target bone tissue is a stable pressure condition, and feeds the stable pressure condition back to the buffer drive portion 230. The buffer drive portion 230 assists the actuator 210 in strengthening the action on the target bone tissue (not shown in the figure), that is, the buffer mechanism drives the actuator 210 to move in the A direction to act on the target bone tissue (not shown in the figure).

Specifically, the stable pressure condition means that a pressure value remains unchanged within a certain period of time or within a certain pressure control range, which indicates that the actuator 210 continuously and stably acts on the target bone tissue. Since the buffer drive portion 230 is fixed to the actuator 210 in a sleeving manner, the buffer drive portion may follow movement of the actuator 210 to stabilize the action on the target bone tissue (not shown in the figure).

**In** some embodiments of the present invention, with reference to Figs. 15 and 16, the actuator 210 acts on the target bone tissue (not shown in the figure) in the A direction shown in Fig. 16, and the pressure sensing portion 220 detects that the change of the pressure by the actuator 210 acting on the target bone tissue is a sudden pressure fall condition, and feeds the sudden pressure fall condition back to the buffer drive portion 230. The buffer drive portion 230 produces reverse thrust in a direction opposite the A direction to weaken the action of the actuator 210 on the target bone tissue (not shown in the figure), thereby reducing the risk of nerve and blood vessel damage due to overshoot during osteotomy.

Specifically, the sudden pressure fall is that the pressure produced by the actuator 210 acting on the target bone tissue is reduced to a minimum value in a first period, that is, that is, the pressure acting on the target bone tissue at the moment when the actuator 210 cuts off the target bone tissue may be attenuated to zero or close to zero, and at this time, it is necessary to reduce the risk of nerve and blood vessel damage by means of the reverse thrust of the buffer drive portion 230 on the actuator 210.

In some embodiments, the minimum value is 0, and the first period is no longer than 0.1 s.

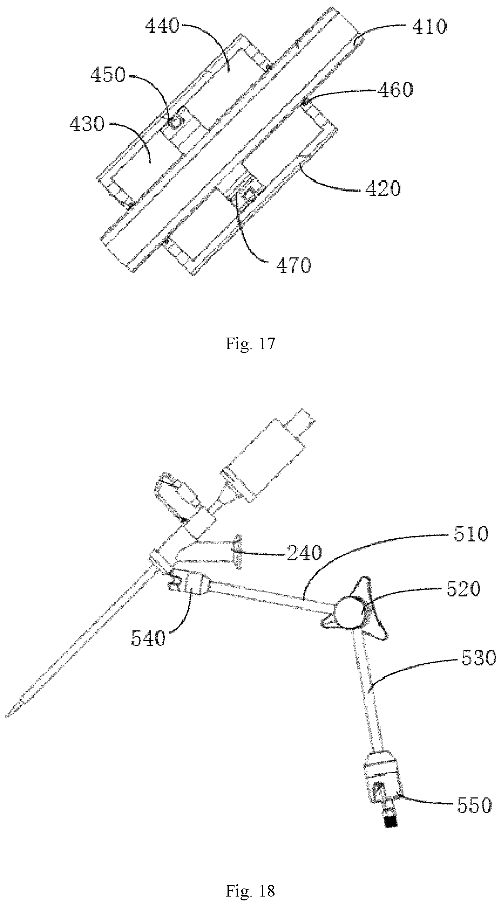

Fig. 17 is a partial structural schematic diagram of a buffer drive portion shown in Fig. 15.

With reference to Figs. 15, 16 and 17, the buffer drive portion 230 includes a closed cavity 420 and a drive portion 410 penetrating the closed cavity 420, the drive portion 410 divides the closed cavity 420 into a first cavity 430 and a second cavity 440, the first cavity 430 is close to an actuation end 250 of the actuator 210, and the second cavity 440 is far away from the actuation end 250 of the actuator 210.

The buffer drive portion 230 further includes a duct portion 330 and an opening and closing control portion (not shown in the figure) which is arranged in the duct portion 330 and in communication connection with the pressure sensing portion 220, the duct portion 330 is arranged in the closed cavity 420 and is in communication connection with the first cavity 430 and the second cavity 440 separately, the opening and closing control portion (not shown in the figure) is composed of a control portion (not shown in the figure) and an opening and closing portion 340 which are in communication connection, and the control portion (not shown in the figure) is in communication connection with the pressure sensing portion 220.

In some specific embodiments, the control portion (not shown in the figure) is a computer control system.

In some specific embodiments, the opening and closing portion 340 is an electromagnetic valve.

In some specific embodiments, the pressure sensing portion 220 is a force sensor.

When the pressure sensing portion 220 detects that the pressure by the actuator 210 acting on the target bone tissue is reduced to the minimum value within the first period as for the change of the pressure by the actuator 210 acting on the target bone tissue, the opening and closing control portion, specifically the control portion, controls the opening and closing portion 340 to close communication of the duct portion 330 with the first cavity 430 and the second cavity 440, such that the drive portion 410 produces the reverse thrust on the actuator 210 in the direction opposite the operation direction to weaken the action of the actuator 210 on the target bone tissue.

Both the first cavity 430 and the second cavity 440 contain hydraulic media. When the opening and closing portion 340 opens the communication of the duct portion 330 with the first cavity 430 and the second cavity 440, the hydraulic media is transferred between the first cavity 430 and the second cavity 440 by means of the duct portion 330.

In some specific embodiments, the hydraulic medium is hydraulic oil.

In some specific embodiments, when the pressure sensing portion 220 detects that the change of the pressure by the actuator 210 acting on the target bone tissue is the stable pressure condition, the buffer drive portion 230 moves along with the actuator 210 in the operation direction to expand a volume in the second cavity 440 and compress a volume in the first cavity 430, in addition, since the volume in the first cavity 430 is compressed, the hydraulic medium therein flows into the second cavity 440 by means of the duct portion 330 to further compress the volume in the first cavity 430, thereby assisting the actuator 210 in moving further in the operation direction to act on the target bone tissue.

In some specific embodiments, when the pressure sensing portion 220 detects and feeds back, to the control portion, that the pressure by the actuator 210 acting on the target bone tissue is reduced to the minimum value within the first period as for the change of the pressure by the actuator 210 acting on the target bone tissue, and the control portion controls the opening and closing portion 340 to close the communication of the duct portion 330 with the first cavity 430 and the second cavity 440, such that the hydraulic medium may not transfer between the first cavity 430 and the second cavity 440.

Just before the pressure produced by the actuator 210 acting on the target bone tissue is reduced to the minimum value within the first period, the hydraulic medium flows into the second cavity 440 by means of the duct portion 330 to further compress the volume in the first cavity 430. At the moment when the hydraulic medium between the first cavity 430 and the second cavity 440 may not be transferred to each other, a pressure in the first cavity 430 is greater than that in the second cavity 440, so as to produce the reverse thrust in the direction opposite the operation direction on the drive portion 410, then push the actuator 210 to move in the direction opposite the operation direction or stop the actuator 210 from moving, and weaken the action of the actuator 210 on the target bone tissue.

Referencing to Fig. 17, the drive portion 410 includes a division portion 470, and an outer side wall of the division portion 470 is attached to an inner side wall of the closed cavity 420, such that division portion moves relative to the closed cavity 420 and divides the closed cavity 420 to form the first cavity 430 and the second cavity 440.

In some embodiments not part of the present invention, the drive portion 410 further includes a hollowed shaft part to penetrate and be fixedly connected to the division portion 470, and penetrate and be movably connected to the closed cavity 420.

In some embodiments not part of the present invention, seal structures are arranged at a joint and an attachment portion between the drive portion 410 and the closed cavity 420.

Not part of the present invention, with reference to Fig. 16 and Fig. 17, a first seal structure 460 is arranged at the joint of the drive portion 410 and the closed cavity 420 to enhance a sealing effect on the closed cavity 420. The outer side wall of the division portion 470 is provided with a second seal structure 450 to attach to the closed cavity 420 and further enhance respective sealing of the first cavity 430 and the second cavity 440.

In some embodiments not part of the present invention, the first seal structure 460 and the second seal structure 450 are both seal rings.

In some embodiments not part of the present invention, the osteotomy device shown in Fig. 15 further includes an observation portion 240 to monitor the action of the actuator 210 on the target bone tissue (not shown in the figure).

With reference to Figs. 15, 16 and 17, not part of the present invention, the observation portion 240 is fixedly connected to the drive portion 410, and the actuator 210 moves through the observation portion 240.

In some embodiments not part of the present invention, the observation portion 240 is an arthroscope with a hollowed structure.

In some embodiments not part of the present invention, the osteotomy device further includes an ultrasonic generator in communication connection with the actuator, such that an actuation end 250 of the actuator 210 acts on the target bone tissue in a high-frequency resonance mode. Specifically, the ultrasonic generator and the actuator 210 together constitute the ultrasonic osteotome.

In some embodiments not part of the present invention, the osteotomy device further includes a movable support portion movably connected to the observation portion 240 to adjust an orientation of the observation portion.

Not part of the present invention, a degree of freedom of motion of the movable support portion is 7, such that the orientation of the observation portion may be adjusted at will.

Not part of the present invention, the movable support portion includes a rotary joint portion and a plurality of ball joint portions.

**In** some embodiments not part of the present invention, the operation system for bone tissue further includes a master control unit and several mechanical arms, where the master control unit is in communication connection with the mechanical arms, and the several mechanical arms are in an identical structure or different structures. The operation system for bone tissue has the beneficial effects as follows: the number of mechanical arms may be increased according to the requirements from implantation, osteotomy or other functional operations, and various use requirements may be met.

Fig. 18 is a structural schematic diagram of another osteotomy device in an embodiment not part of the present invention.

The osteotomy device shown in Fig. 18 differs from the osteotomy device shown in Fig. 15 in that the osteotomy device shown in Fig. 18 further includes a rotary joint portion consisting of a first rod portion 510 and a second rod portion 530 which are movably connected, and a first ball joint portion 540 and a second ball joint portion 550 which are arranged at two ends of the rotary joint portion, and the first ball joint portion 540 is movably connected to the observation portion 240 to adjust the orientation of the observation portion 240. Not part of the present invention.

**In** some embodiments not part of the present invention, a joint location of the rotary joint is movably connected to a locking portion for adjusting a relative location between the rotary joint portion and the observation portion 240.

Not part of the present invention, with reference to Fig. 18, the locking portion 520 is provided at the joint of the first rod portion 510 and the second rod portion 530.

Operation steps of the osteotomy device in the embodiment of the present disclosure are described below with reference to Figs. 3, 15 and 18.

After the actuator 210 is introduced into the vicinity of the vertebral pedicle 21 to be broken, the locking portion 520 is not locked, such that the observation portion 240 and the actuator 210 may move flexibly to find a location where osteotomy is required.

Then, locations, relative to the movable support (not shown in the figure), of the observation portion 240 and the actuator 210 are fixed by the locking portion 520, and finally, the osteotomy is performed by the actuator 210 with assistance of the observation portion 240.

Although the embodiments of the present disclosure have been described in detail above, it is apparent to those skilled in the art that various modifications and changes can be made to these embodiments. However, it should be understood that such modifications and changes are within the scope of appended claims.

## Claims

1. An operation system for bone tissue (1), comprising
a first implantation device (13) and a second implantation device (14), wherein the first implantation device comprises a hollowed structure, an adapter structure is arranged in the hollowed structure, and at least a portion of an outer side wall of a bottom of the second implantation device is provided with a corresponding adapter structure to be fixedly connected to the adapter structure, wherein the operation system further comprises an osteotomy device (132) for acting on target bone tissue to form a space, a master control unit and an osteotomy mechanical arm (131) clamping the osteotomy device, wherein the master control unit is in communication connection with the osteotomy mechanical arm, and the osteotomy device comprises an actuator (210) clamped at a free end of the osteotomy mechanical arm, and the master control unit controls the osteotomy mechanical arm to cause the actuator to act on the target bone tissue to form the space,
**characterized in that**
the osteotomy mechanical arm further comprises a buffer mechanism partially sleeving the actuator, the buffer mechanism comprises a closed cavity (420) and a drive portion (410) penetrating the closed cavity, the drive portion divides the closed cavity into a first cavity (430) and a second cavity (440), the actuator is sleeved with the drive portion, the first cavity is close to an actuation end of the actuator, and the second cavity is far away from the actuation end of the actuator;
the drive portion (410) includes a division portion (470), and an outer side wall of the division portion (470) is attached to an inner side wall of the closed cavity (420), such that division portion moves relative to the closed cavity (420) and divides the closed cavity (420) to form the first cavity (430) and the second cavity (440);
the buffer mechanism further comprises a pressure sensing portion (220) arranged on the actuator to acquire change of a pressure by the actuator acting on the target bone tissue;
the buffer mechanism further comprises a duct portion (330) which is arranged in the closed cavity and is in communication with the first cavity (430) and the second cavity (440) separately;
both the first cavity (430) and the second cavity (440) contain hydraulic media;
the hydraulic media is transferred between the first cavity (430) and the second cavity (440) by means of the duct portion (330);
the duct portion is provided with an opening and closing control portion which is in communication connection with the pressure sensing portion (220); and
when the pressure sensing portion (220) detects that the pressure by the actuator acting on the target bone tissue is reduced to a minimum value within a first period as for the change of the pressure by the actuator acting on the target bone tissue, the opening and closing control portion closes communication of the duct portion (330) with the first cavity and the second cavity, such that the drive portion (410) produces a reverse thrust on the actuator in a direction opposite an operation direction to weaken the action of the actuator on the target bone tissue.

## Patentansprüche

1. Operationssystem für Knochengewebe (1), umfassend
eine erste Implantationsvorrichtung (13) und eine zweite Implantationsvorrichtung (14), wobei die erste Implantationsvorrichtung eine ausgehöhlte Struktur umfasst, eine Adapterstruktur in der ausgehöhlten Struktur angeordnet ist und mindestens ein Abschnitt einer äußeren Seitenwand einer Unterseite der zweiten Implantationsvorrichtung mit einer entsprechenden Adapterstruktur versehen ist, um fest mit der Adapterstruktur verbunden zu werden, wobei das Operationssystem ferner eine Osteotomievorrichtung (132) umfasst, um auf das Zielknochengewebe zu wirken, um einen Raum zu bilden, eine Hauptsteuereinheit und einen mechanischen Osteotomiearm (131), der die Osteotomievorrichtung klemmt, wobei die Hauptsteuereinheit in Kommunikationsverbindung mit dem mechanischen Osteotomiearm ist und die Osteotomievorrichtung einen Aktuator (210) umfasst, der an einem freien Ende des mechanischen Osteotomiearms geklemmt ist und die Hauptsteuereinheit den mechanischen Osteotomiearm steuert, um den Aktuator zu veranlassen, auf das Zielknochengewebe zu wirken, um den Raum zu bilden,
**dadurch gekennzeichnet, dass**
der mechanische Osteotomiearm ferner einen Puffermechanismus umfasst, der den Aktuator teilweise ummantelt, der Puffermechanismus einen geschlossenen Hohlraum (420) und einen Antriebsabschnitt (410) umfasst, der in den geschlossenen Hohlraum eindringt, der Antriebsabschnitt den geschlossenen Hohlraum in einen ersten Hohlraum (430) und einen zweiten Hohlraum (440) teilt, der Aktuator mit dem Antriebsabschnitt ummantelt ist, der erste Hohlraum sich nahe an einem Betätigungsende des Aktuators befindet und der zweite Hohlraum sich weit entfernt vom Betätigungsende des Aktuators befindet;
der Antriebsabschnitt (410) einen Teilungsabschnitt (470) einschließt und eine äußere Seitenwand des Teilungsabschnitts (470) an einer inneren Seitenwand des geschlossenen Hohlraums (420) angebracht ist, sodass sich der Teilungsabschnitt relativ zum geschlossenen Hohlraum (420) bewegt und den geschlossenen Hohlraum (420) teilt, um den ersten Hohlraum (430) und den zweiten Hohlraum (440) zu bilden;
der Puffermechanismus ferner einen Druckerfassungsabschnitt (220) umfasst, der auf dem Aktuator angeordnet ist, um eine Druckänderung durch den Aktuator, der auf das Zielknochengewebe wirkt, zu erfassen;
der Puffermechanismus ferner einen Leitungsabschnitt (330) umfasst, der im geschlossenen Hohlraum angeordnet ist und getrennt mit dem ersten Hohlraum (430) und dem zweiten Hohlraum (440) in Kommunikation ist;
sowohl der erste Hohlraum (430) als auch der zweite Hohlraum (440) hydraulische Medien enthalten;
die hydraulischen Medien zwischen dem ersten Hohlraum (430) und dem zweiten Hohlraum (440) mittels des Leitungsabschnitts (330) übertragen werden;
der Leitungsabschnitt mit einem Öffnungs- und Schließsteuerabschnitt versehen ist, der in Kommunikationsverbindung mit dem Druckerfassungsabschnitt (220) ist, und
wenn der Druckerfassungsabschnitt (220) erkennt, dass der Druck durch den auf das Zielknochengewebe wirkenden Aktuator innerhalb eines ersten Zeitraums auf einen Mindestwert reduziert wird aufgrund der Änderung des Drucks durch den auf das Zielknochengewebe wirkenden Aktuator, schließt der Öffnungs- und Schließsteuerabschnitt die Kommunikation des Leitungsabschnitts (330) mit dem ersten Hohlraum und dem zweiten Hohlraum, sodass der Antriebsabschnitt (410) einen Rückwärtsschub auf den Aktuator in einer einer Operationsrichtung entgegengesetzten Richtung erzeugt, um die Wirkung des Aktuators auf das Zielknochengewebe zu schwächen.

## Revendications

1. Système de chirurgie pour tissu osseux (1), comprenant
un premier dispositif d'implant (13) et un deuxième dispositif d'implant (14), dans lequel le premier dispositif d'implant comprend une structure creuse, une structure adaptative est disposée dans la structure creuse, et au moins une partie d'une paroi latérale externe d'un fond du deuxième dispositif d'implant est pourvue d'une structure adaptative correspondante pour être reliée de manière fixe à la structure adaptative, dans lequel le système de chirurgie comprend en outre un dispositif d'ostéotomie (132) destiné à agir sur un tissu osseux cible pour former un espace, une unité de commande maître et un bras mécanique d'ostéotomie (131) serrant le dispositif d'ostéotomie, dans lequel l'unité de commande maître est relié en communication avec le bras mécanique d'ostéotomie, et le dispositif d' ostéotomie comprend un actionneur (210) serré à une extrémité libre du bras mécanique d'ostéotomie, et l'unité de commande maître commande le bras mécanique d'ostéotomie pour amener l'actionneur à agir sur le tissu osseux cible pour former l'espace,
**caractérisé en ce que**
le bras mécanique d'ostéotomie comprend en outre un mécanisme tampon emmanchant l'actionneur partiellement, le mécanisme tampon comprend une cavité fermée (420) et une partie d'entraînement (410) pénétrant dans la cavité fermée, la partie d'entraînement divise la cavité fermée en une première cavité (430) et une deuxième cavité (440), l'actionneur est emmanché avec la partie d'entraînement, la première cavité est proche d'une extrémité d'actionnement de l'actionneur, et la deuxième cavité est éloignée de l'extrémité d'actionnement de l'actionneur ;
la partie d'entraînement (410) comporte une partie de division (470), et une paroi latérale externe de la partie de division (470) est attachée à une paroi latérale interne de la cavité fermée (420), de telle sorte que la partie de division se déplace par rapport à la cavité fermée (420) et divise la cavité fermée (420) pour former la première cavité (430) et la deuxième cavité (440) ;
le mécanisme tampon comprend en outre une partie de détection de pression (220) disposée sur l'actionneur pour acquérir un changement d'une pression par l'actionneur agissant sur le tissu osseux cible ;
le mécanisme tampon comprend en outre une partie de conduit (330) qui est disposée dans la cavité fermée et est en communication avec la première cavité (430) et la deuxième cavité (440) séparément ;
chacune de la première cavité (430) et de la deuxième cavité (440) contient un milieu hydraulique ;
le milieu hydraulique est transféré entre la première cavité (430) et la deuxième cavité (440) au moyen de la partie de conduit (330) ;
la partie de conduit est pourvue d'une partie de commande d'ouverture et de fermeture qui est reliée en communication avec la partie de détection de pression (220) ; et
lorsque la partie de détection de pression (220) détecte que la pression exercée par l'actionneur agissant sur le tissu osseux cible est réduite à une valeur minimale au cours d'une première période comme pour le changement de la pression par l'actionneur agissant sur le tissu osseux cible, la partie de commande d'ouverture et de fermeture ferme la communication de la partie de conduit (330) avec la première cavité et la deuxième cavité, de telle sorte que la partie d'entraînement (410) produise une poussée inverse sur l'actionneur dans une direction opposée à une direction de fonctionnement pour affaiblir l'action de l'actionneur sur le tissu osseux cible.
